# EUROPEAN PATENT SPECIFICATION

(11) **EP 2 222 293 B1**
(45) Date of publication and mention of the grant of the patent: **03.08.2011**
(21) Application number: 08857026.2
(22) Date of filing: 03.12.2008
(51) Int. Cl.: A61K 31/205, A61P 9/00, A61P 9/10

(54) **MEDICAL USE OF 3-(2,2,2-TRIMETHYLHYDRAZINIUM) PROPIONATE HYDROGEN FUMARATE AND DIHYDROGEN PHOSPHATE**
MEDIZINISCHE VERWENDUNG VON 3-(2,2,2-TRIMETHYLHYDRAZINIUM) PROPIONATHYDROGENFUMARAT UND DIHYDROGENPHOSPHAT
NOUVEL USAGE MÉDICAL DES SELS DE 3-(2,2,2-TRIMÉTHYLHYDRAZINIUM) PROPIONATE

(30) Priority: 04.12.2007 EP 07122272; 04.12.2007 EP 07122273
(43) Date of publication of application: 01.09.2010
(73) Proprietor: Grindeks, a joint stock company, Riga 1057 (LV)
(72) Inventor: STONANS, Ilmars, LV-1057 Riga (LV); TARARAK, Eduard, Moscow 121609 (RU); ANDREYEVA, Elena, Moscow 121614 (RU)
(86) International application number: PCT/EP2008/066711
(87) International publication number: WO 2009/071585

(56) References cited:
- WO-A-2005/012233
- KARPOV R S ET AL: "CLINICAL AND INSTRUMENT ASSESSMENT OF THE EFFICACY OF TREATING PATIENTS WITH COMBINED ATHEROSCLEROSIS OF THE CORONARY CEREBRAL AND PERIPHERAL ARTERIES" TERAPEVTICHESKII ARKHIV, vol. 63, no. 4, 1991, pages 90-93, XP008087626 ISSN: 0040-3660
- OKUNEVICH I V ET AL: "[Anti-atherosclerotic action of mildronate in experiment]" PATOLOGICHESKAIA FIZIOLOGIIA I ÈKSPERIMENTAL'NAIA TERAPIIA 2002 APR-JUN, no. 2, April 2002 (2002-04), pages 24-27, XP008087617 ISSN: 0031-2991
- VEVERIS M ET AL: "Experimental hypercholesterolemia and atherosclerosis model in laboratory mice" BALTIC JOURNAL OF LABORATORY ANIMAL SCIENCE, vol. 10, no. 3-4, 2000, pages 194-199, XP008087675 ISSN: 1407-0944

## Description

### Technical Field

The present invention relates to the used 3-(2,2,2-trimethylhydrazinium) propionate salts selected from the group of dihydrogen phosphate and hydrogen fumarate for the manufacture of a medicament for prevention and therapy of atherosclerosis.

### Background Art

Atherosclerosis is a disease affecting arterial blood vessels. It is a chronic inflammatory response in the walls of arteries, in large part due to the deposition of lipoproteins (plasma proteins that carry cholesterol and triglycerides). It is commonly referred to as a "hardening" or "furring" of the arteries. It is caused by the formation of multiple plaques within the arteries.

Atherosclerosis is a slow, complex disease that typically starts in childhood and often progresses with age. In some people it progresses rapidly, even in their third decade. The disease is thought to start from damage to the innermost layer of the artery which is called the endothelium. The damage to the arterial wall is caused by:
- elevated levels of cholesterol and triglyceride (tri-GLIS'er-id) in the blood
- high blood pressure
- tobacco smoke
- diabetes

See, http://www.americanheart.org/presenter.jhtml?identifier=4440 04.12.2007 Atherosclerosis is a great social and medical-sociologic problem and its clinical manifestations are the major contributor to high hospitalisation and death rates. 3-(2,2,2-trimethylhydrazinium) propionate dihydrate is known under International Nonproprietary Name-Meldonium dihydrate.

Carnitine and Meldonium are structurally very similar.

Carnitine has been used as an anti-atherosclerosis agent, alone or in combination with other drugs, preferably naturally occurring preparates, such as flavonoids or omega-3 series polyunsaturated acids, etc.

Patent EP1128822 (SIGMA TAU HEALTHSCIENCE SPA, published in 05.09.2001) discloses a pharmaceutical composition comprising L-carnitine and a flavonoid against thrombosis and atherosclerosis.

However, the pharmacological effect of Meldonium dihydrate has been regarded as counteracting the effect of carnitine.

Meldonium dihydrate has been used in anti-atherosclerosis therapy. Meldonium dihydrate displays hypolipodemic activity in rats with triton WR-1339 hyperlipidemia. (OKUNEVICH IV, RYZHENKOV VE et al, "Anti-atherosclerotic action of Meldonium dihydrate in experiment", published in "Patologiteskaja Fiziologija I Experimentaljnaja Terapija" 2002, Apr-Jun vol.2, p.24-7).

An experiment that describes atherosclerosis in coronary, cerebral and peripheral blood-vessels shows that Meldonium dihydrate exerts a beneficial effect on the regional circulation, lipid metabolism and can be used in the treatment of patients with concomitant forms of atherosclerosis (see KARPOV RS, DUDKO VA. "The clinical instrumental evaluation of treatment efficacy in patients with concomitant atherosclerosis of the coronary, cerebral and peripheral arteries", published in "Terapevticheskii Arkhiv" 1991. vol.63, no.4, p.90-93).

Dihydrogen phosphate and hydrogen fumarate salts of Meldonium are disclosed in EP 1667960 A (JOINT STOCK COMPANY GRINDEKS) 14.06.2006 as more stable substance comparatively with Meldonium dihydrate.

### Disclosure of Invention

### Technical problem

Irrespective of a vast range of pharmacological preparations used to treat patients with atherosclerosis, the disease if not dully controlled.

The aim of the present invention is to get a pharmaceutical substance which is more effective substance than Meldonium dihydrate in prevention and therapy of atherosclerosis.

We unexpectedly have found that salts of Meldonium - Meldonium dihydrogen phosphate and Meldonium hydrogen fumarate, effectively reduce atherosclerotic manifestations in the aorta.

### Anti-Atherosclerotic experiment

Pharmacological experiment was carried out to examine the anti-atherosclerotic effect of Meldonium dihydrogen phosphate or Meldonium hydrogen fumarate in the aorta.

The experiments with Meldonium salt such as Meldonium dihydrogen phosphate or Meldonium hydrogen fumarate, were performed in 38 Chinchilla rabbits weighing 2.5-3.0 kg. The rabbits were randomly divided into 4 groups: Control (n=11), Meldonium dihydrate group (n=9) and Meldonium dihydrogen phosphate group (n=9) and Meldonium hydrogen fumarate group (n=9).

Experimental atherosclerosis, i.e. serum hyperlipidaemia and the atherosclerotic manifestations in the aorta, was induced by administering 1% cholesterol by weight together with standard diet.

The rabbits were fed with standard diet with 1% cholesterol dissolved in the sunflower seed oil (4% from the total amount of food). The diet was enriched with cholesterol as follows: 10 kg standard food for rabbits was heated to 60°C and was mixed with 100 g warm cholesterol dissolved in 400 ml sunflower seed oil. The rabbits received 200 g of this food per day.

The experimental groups were given Meldonium dihydrate, Meldonium dihydrogen phosphate and Meldonium hydrogen fumarate, in a dose 124 mg/kg, 165 mg/kg and 174 mg/kg. The preparations were added to drinking water.

After 15 weeks the rabbits were sacrificed. Heart, aorta and liver were collected for examination. The aorta was stripped of adventitia, cut longitudinally and washed with phosphate buffer solution. Specimens from abdominal, descending and ascending aorta were studied by light microscopy and immunomorphologically. Pieces of aorta were fixed in 4% neutral formaldehyde buffer for 7 days. For further investigations the aorta, heart and liver were frozen in liquid nitrogen and stored at 70°C. Specimens were fixed in methacarn for preparation of paraffin sections.

After fixation in formaldehyde the specimens were washed for 24 hours in running water, stained with Oil red O by the standard method and examined for the presence of lipid spots, streaks and plaques.

In all rabbits atherosclerotic changes were observed in the total preparations of the aorta stained with Oil red O.

Results of these tests show that and Meldonium dihydrogen phosphate is capable of reducing the spots of atherosclerosis manifestations, see Table 1. Anti-Atherosclerotic activity of Meldonium dihydrogen phosphate

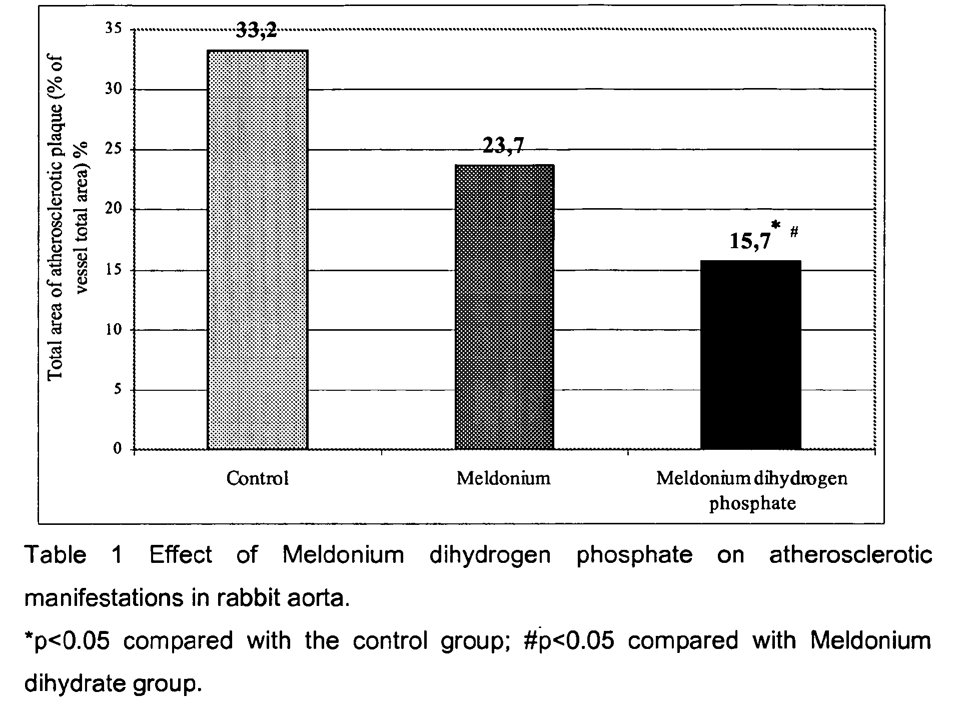

Table 1 shows that Meldonium dihydrogen phosphate in pharmacological reduces >2-fold the area of atherosclerotic plaque in rabbit aorta compared with control and Meldonium dihydrate groups.

The results of these tests show that Meldonium's hydrogen fumarate reduces atherosclerosis manifestations see Table 2.

### Anti-Atherosclerotic activity of Meldonium hydrogen fumarate

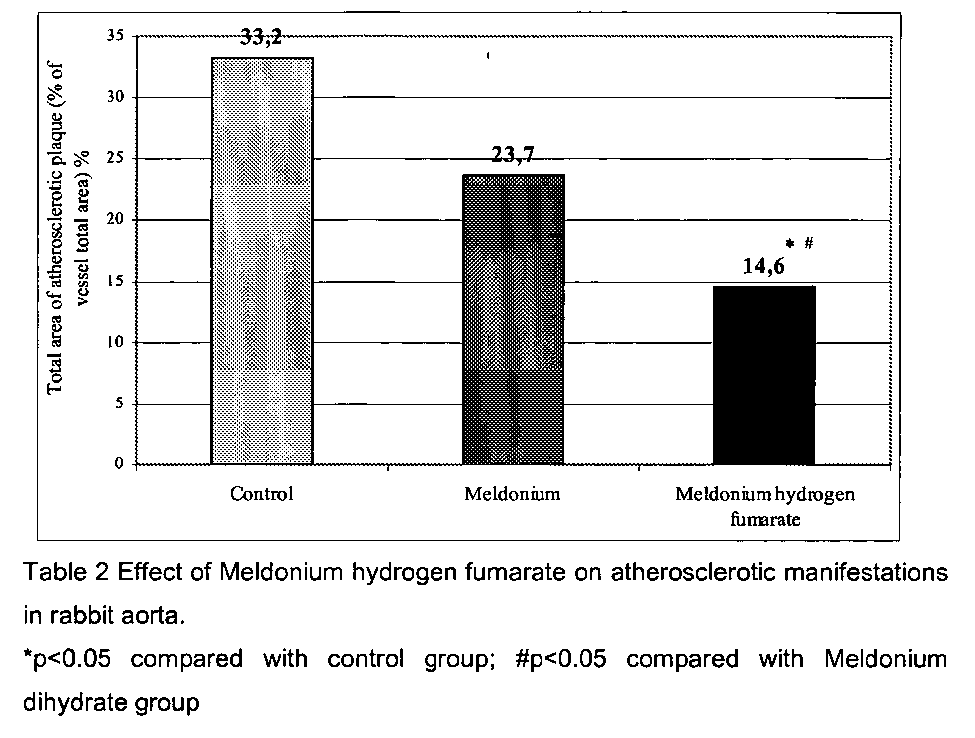

Table 2 shows that Meldonium's hydrogen fumarate in pharmacological doses reduce >2-fold the area of atherosclerotic plaque in rabbit aorta compared with the control and Meldonium dihydrate groups

## Claims

1. Use of 3-(2,2,2-trimethylhydrazinium) propionate salt selected from the group consisting of dihydrogen phosphate and hydrogen fumarate for manufacture of a medicament for prophylaxis and/or treatment of atherosclerosis.

2. Use according to claim 1, wherein 3-(2,2,2-trimethylhydrazinium) propionate salt is 3-(2,2,2-trimthylhydrazinium) propionate dihydrogen phosphate.

3. Use according to claim 1, wherein 3-(2,2,2-trimethylhydrazinium) propionate salt is 3-(2,2,2-trimthylhydrazinium) propionate hydrogen fumarate.

4. 3-(2,2,2-Trimethylhydrazinium) propionate salt selected from the group consisting of dihydrogen phosphate and hydrogen fumarate for use in the prophylaxis and/or treatment of atherosclerosis.

## Patentansprüche

1. Anwenung von 3-(2,2,2-trimethylhydrazinium)propionatsalz, ausgewählt aus einer Gruppe, zu der Dihydrogenphosphat und Hydrogenfumarat gehören, für die Vorbeugung und/oder Produktion von Arzneimittel für die Behandlung von Arteriosklerose.

2. Anwendung nach Anspruch 1, soweit 3-(2,2,2-trimethylhydrazinium)propionatsalz das 3-(2,2,2-trimethylhydrazinium)propionat-dihydrogenphosphat ist.

3. Anwendung nach Anspruch 1, soweit 3-(2,2,2-trimethylhydrazinium)propionatsalz das 3-(2,2,2-trimethylhydrazinium)propionat-hydrogenfumarat ist.

4. 3-(2,2,2-trimethylhydrazinium)propionatsalz, ausgewählt aus einer Gruppe, zu der Dihydrogenphosphat und Hydrogenfumarat gehören, für die Vorbeugung und/oder Behandlung von Arteriosklerose.

## Revendications

1. Utiliser du sel 3-(2,2,2-trimethylhydrazinium) propionate choisi du groupe consistant de dihydrogénophosphate et hydrogène fumarate pour fabriquer le médicament pour prophylaxie et/ou traitement d'athérosclérose.

2. Utiliser selon la réclamation 1, où le sel 3-(2,2,2-trimethylhydrazinium) propionate est 3-(2,2,2-trimthylhydrazinium) propionate dihydrogénophosphate.

3. Utiliser selon la réclamation 1, où le sel 3-(2,2,2-trimethylhydrazinium) propionate est 3-(2,2,2-trimthylhydrazinium) propionate hydrogène fumarate.

4. Le sel 3-(2,2,2-Trimethylhydrazinium) propionate choisi du groupe consistant de dihydrogénophosphate et hydrogène fumarate pour la prophylaxie et/ou traitement d'athérosclérose
